# EUROPEAN PATENT APPLICATION

(11) **EP 4 660 187 A1**
(43) Date of publication of application: **10.12.2025**
(21) Application number: 24750019.2
(22) Date of filing: 22.01.2024
(51) Int. Cl.: C07C 257/12, C07F 5/00, C23C 16/06, C23C 16/34, C23C 16/40

(54) **COMPOUND, RAW MATERIAL FOR THIN FILM FORMATION, THIN FILM, AND METHOD FOR PRODUCING THIN FILM**

(30) Priority: 01.02.2023 JP 2023013693
(71) Applicant: ADEKA CORPORATION, Arakawa-ku Tokyo 116-8554 (JP)
(72) Inventor: HATASE, Masako, Tokyo 116-8554 (JP); SAKURAI, Atsushi, Tokyo 116-8554 (JP); FUSE, Wakana, Tokyo 116-8554 (JP); HARANO, Kazuki, Tokyo 116-8554 (JP); MURATA, Kiyoshi, Tokyo 116-8554 (JP); NISHIDA, Akihiro, Tokyo 116-8554 (JP); FUKUSHIMA, Ryota, Tokyo 116-8554 (JP)
(74) Representative: Müller-Boré & Partner Patentanwälte PartG mbB
(86) International application number: PCT/JP2024/001654
(87) International publication number: WO 2024/162067

(57) **Abstract**

This disclosure provides a compound represented by the following general formula (1), a thin-film forming raw material containing the compound, a thin-film formed from the thin-film forming raw material, and a method of producing a thin-film: 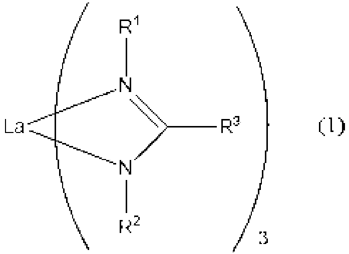 where R¹ and R² each independently represent an alkyl group having 1 to 8 carbon atoms, and R³ represents a hydrogen atom or an alkyl group having 1 to 5 carbon atoms, provided that R¹ and R² represent different groups.

## Description

### Technical Field

The present invention relates to a compound having a specific structure, a thin-film forming raw material, a thin-film formed from a thin-film forming raw material, and a method of producing a thin-film.

### Background Art

A thin-film forming raw material containing a lanthanum atom is used as a material for producing a DRAM gate, a logic transistor, and the like.

As the above-mentioned method of producing a thin-film, there are given, for example, a sputtering method, an ion plating method, a MOD method, such as a coating thermal decomposition method or a sol-gel method, and a chemical vapor deposition method. However, a chemical vapor deposition (hereinafter sometimes simply referred to as "CVD") method including an atomic layer deposition (hereinafter sometimes simply referred to as "ALD") method is an optimum production process because the method has many advantages, such as excellent composition controllability and step coverage, suitability for mass production, and capability of hybrid integration.

A large number of various raw materials have been reported as a source for supplying a lanthanum element to be used in the chemical vapor deposition method. For example, in Patent Literature 1, there is a disclosure of a lanthanum compound coordinated with a ketoimine ligand and an alkoxy group, and in Patent Literature 2, there is a disclosure of a lanthanum amidinate. In addition, in Patent Literature 3, there is a disclosure of a lanthanum compound coordinated with an N-heterocyclic carbene and an alkoxy group.

### Citation List

### Patent Literature

[PTL 1] JP 2012-153688 A
[PTL 2] JP 2017-122273 A
[PTL 3] JP 2022-077508 A

### Summary of Invention

### Technical Problem

However, when a thin-film is produced through use of each of the compounds described in Patent Literatures 1 to 3, there has been a problem in that it is difficult to obtain a high-quality thin-film containing a lanthanum atom, the thin-film having a small residual carbon amount.

Accordingly, an object of the present invention is to provide a thin-film forming raw material capable of producing a high-quality thin-film containing a lanthanum atom (hereinafter sometimes referred to as "lanthanum-containing thin-film"), the thin-film having a small residual carbon amount, and a method of producing a thin-film using the same.

### Solution to Problem

The inventors of the present invention have made extensive investigations, and as a result, have found that the use of a compound having a specific structure can solve the above-mentioned problems. Thus, the inventors have completed the present invention.

That is, according to a first aspect of the present invention, there is provided a compound represented by the following general formula (1): 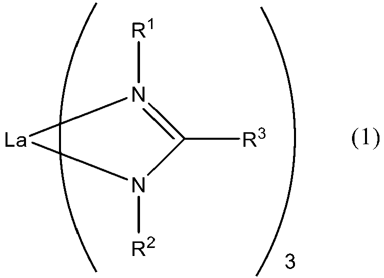 where R¹ and R² each independently represent an alkyl group having 1 to 8 carbon atoms, and R³ represents a hydrogen atom or an alkyl group having 1 to 5 carbon atoms, provided that R¹ and R² represent different groups.

According to a second aspect of the present invention, there is provided a thin-film forming raw material, comprising the above-mentioned compound.

According to a third aspect of the present invention, there is provided a thin-film formed from the above-mentioned thin-film forming raw material.

According to a fourth aspect of the present invention, there is provided a method of producing a thin-film, comprising forming a thin-film containing a lanthanum atom on a surface of a substrate through use of the above-mentioned thin-film forming raw material.

A compound represented by the following general formula (2) is synonymous with the compound represented by the general formula (1): 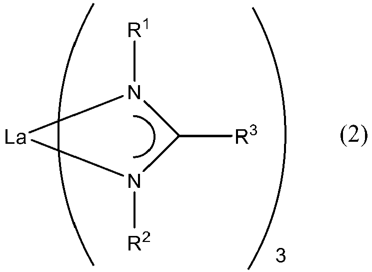 where R¹ and R² each independently represent an alkyl group having 1 to 8 carbon atoms, and R³ represents a hydrogen atom or an alkyl group having 1 to 5 carbon atoms, provided that R¹ and R² represent different groups.

### Advantageous Effects of Invention

According to the compound disclosed herein, the thin-film forming raw material capable of producing a high-quality lanthanum-containing thin-film having a small residual carbon amount, and the method of producing a thin-film using the same can be provided.

### Brief Description of Drawings

FIG. 1 is a schematic diagram for illustrating an example of an ALD apparatus to be used in a method of producing a thin-film according to a fourth aspect of the present invention.
FIG. 2 is a schematic diagram for illustrating another example of the ALD apparatus to be used in the method of producing a thin-film according to the fourth aspect of the present invention.
FIG. 3 a schematic diagram for illustrating still another example of the ALD apparatus to be used in the method of producing a thin-film according to the fourth aspect of the present invention.
FIG. 4 is a schematic diagram for illustrating yet still another example of the ALD apparatus to be used in the method of producing a thin-film according to the fourth aspect of the present invention.

### Description of Embodiments

### [Compound]

A compound according to a first aspect of the present invention is a compound represented by the following general formula (1).

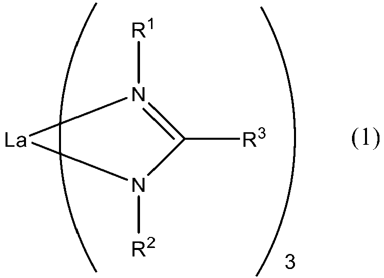

In the general formula (1), R¹ and R² each independently represent an alkyl group having 1 to 8 carbon atoms, and R³ represents a hydrogen atom or an alkyl group having 1 to 5 carbon atoms, provided that R¹ and R² represent different groups.

Examples of the alkyl group having 1 to 8 carbon atoms represented by each of R¹ and R² include a methyl group, an ethyl group, a n-propyl group, an isopropyl group, a n-butyl group, an isobutyl group, a sec-butyl group, a tert-butyl group, a n-pentyl group, an isopentyl group, a sec-pentyl group (2-pentyl group), a 3-pentyl group, a neopentyl group, a n-hexyl group, an isohexyl group, a sec-hexyl group, a tert-hexyl group, a n-heptyl group, an isoheptyl group, a sec-heptyl group, a tert-heptyl group, a n-octyl group, an isooctyl group, a sec-heptyl group, a tert-octyl group, and a 2-ethylhexyl group.

Herein, the term "sec-pentyl" refers to 2-pentyl.

Examples of the alkyl group having 1 to 5 carbon atoms represented by R³ include a methyl group, an ethyl group, a n-propyl group, an isopropyl group, a n-butyl group, an isobutyl group, a sec-butyl group, a tert-butyl group, a n-pentyl group, an isopentyl group, and a neopentyl group.

From the viewpoint of providing a compound having a low melting point and a high vapor pressure, and the viewpoint of providing a thin-film forming raw material capable of producing a high-quality lanthanum-containing thin-film having a small residual carbon amount with higher productivity, R¹ and R² each represent preferably an alkyl group having 1 to 6 carbon atoms, more preferably an alkyl group having 3 to 6 carbon atoms, still more preferably a branched alkyl group having 3 to 6 carbon atoms, particularly preferably any one of an isopropyl group, a sec-butyl group, a sec-pentyl group (2-pentyl group), and a 3-pentyl group. From the viewpoint of providing a compound having a low melting point and a high vapor pressure, and the viewpoint of providing a thin-film forming raw material capable of producing a high-quality lanthanum-containing thin-film having a small residual carbon amount with higher productivity, the combination of R¹ and R² is preferably such a combination that both of R¹ and R² represent groups selected from alkyl groups each having 3 to 6 carbon atoms, more preferably such a combination that both of R¹ and R² represent groups selected from branched alkyl groups each having 3 to 6 carbon atoms, still more preferably such a combination that any one of R¹ and R² represents a group selected from a sec-butyl group, a sec-pentyl group (2-pentyl group), and 3-pentyl group, and the other represents an isopropyl group, particularly preferably such a combination that any one of R¹ and R² represents a group selected from a sec-butyl group and a sec-pentyl group (2-pentyl group), and the other represents an isopropyl group, most preferably such a combination that any one of R¹ and R² represents a sec-pentyl group (2-pentyl group), and the other represents an isopropyl group.

From the viewpoint of providing a compound having a low melting point and a high vapor pressure, and the viewpoint of providing a thin-film forming raw material capable of producing a high-quality lanthanum-containing thin-film having a small residual carbon amount with higher productivity, R³ represents preferably a hydrogen atom or an alkyl group having 1 to 3 carbon atoms, more preferably a hydrogen atom or a methyl group, particularly preferably a hydrogen atom.

From the viewpoint of providing a compound having a low melting point and a high vapor pressure, and the viewpoint of providing a thin-film forming raw material capable of producing a high-quality lanthanum-containing thin-film having a small residual carbon amount with higher productivity, when R³ represents a hydrogen atom or a methyl group, preferably when R³ represents a hydrogen atom, the combination of R¹ and R² is preferably such a combination that both of R¹ and R² represent groups selected from alkyl groups each having 3 to 6 carbon atoms, more preferably such a combination that both of R¹ and R² represent groups selected from branched alkyl groups each having 3 to 6 carbon atoms, still more preferably such a combination that any one of R¹ and R² represents a group selected from a sec-butyl group, a sec-pentyl group (2-pentyl group), and a 3-pentyl group, and the other represents an isopropyl group, particularly preferably such a combination that any one of R¹ and R² represents a group selected from a sec-butyl group and a sec-pentyl group (2-pentyl group), and the other represents an isopropyl group, most preferably such a combination that any one of R¹ and R² represents a sec-pentyl group (2-pentyl group), and the other represents an isopropyl group.

Preferred specific examples of the compound represented by the general formula (1) to be used in the thin-film forming raw material of the present invention include Compounds No. 1 to No. 120 below, but the present invention is not limited to these compounds. In the following chemical formulae, "Me" represents a methyl group, "Et" represents an ethyl group, "nPr" represents a n-propyl group, "iPr" represents an isopropyl group, "nBu" represents a n-butyl group, "iBu" represents an isobutyl group, "sBu" represents a sec-butyl group, "tBu" represents a tert-butyl group, "nPe" represents a n-pentyl group, "iPe" represents an isopentyl group, "sPe" represents a sec-pentyl group (2-pentyl group), and "3Pe" represents a 3-pentyl group.

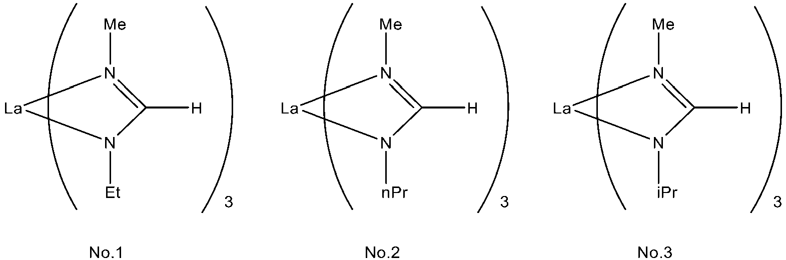

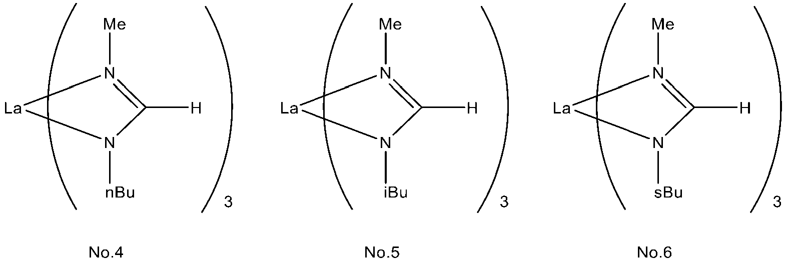

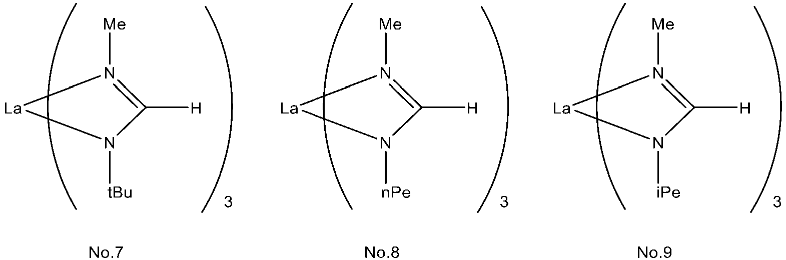

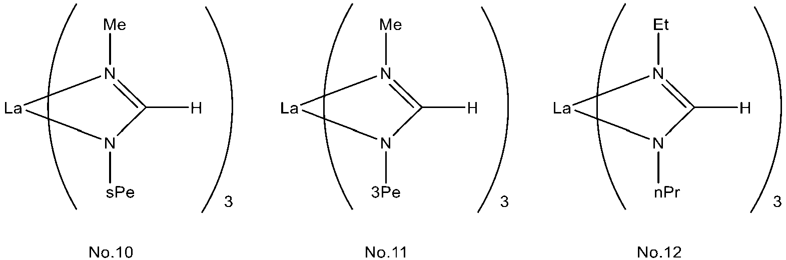

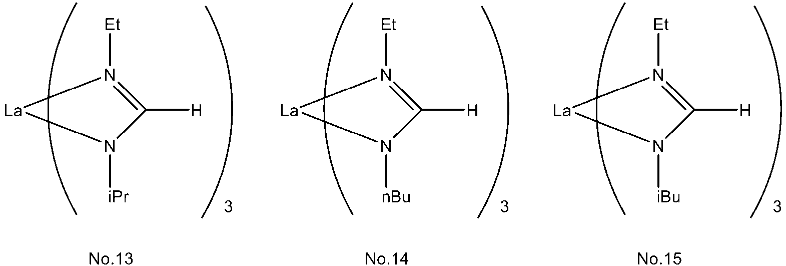

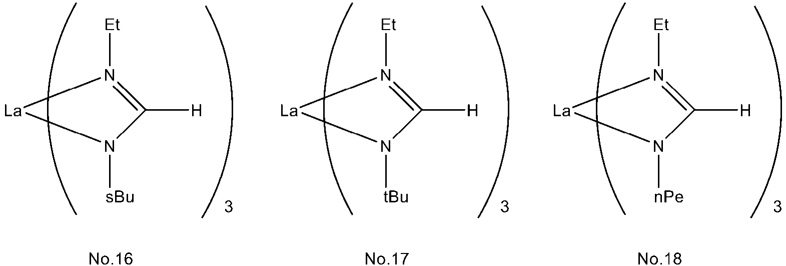

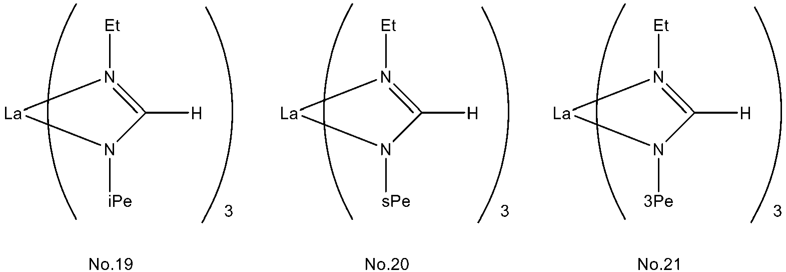

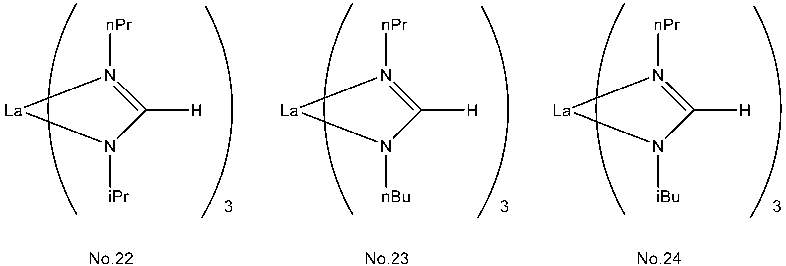

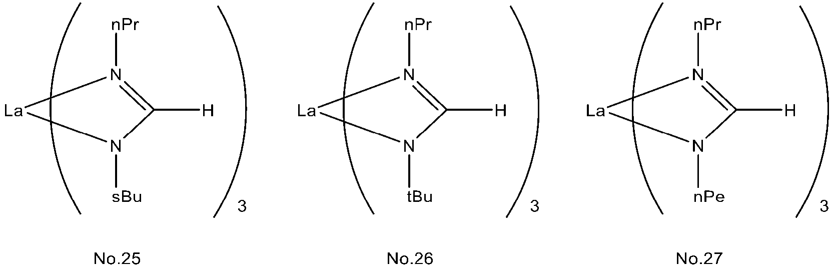

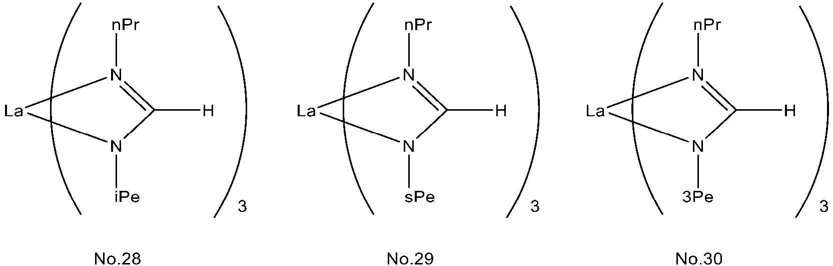

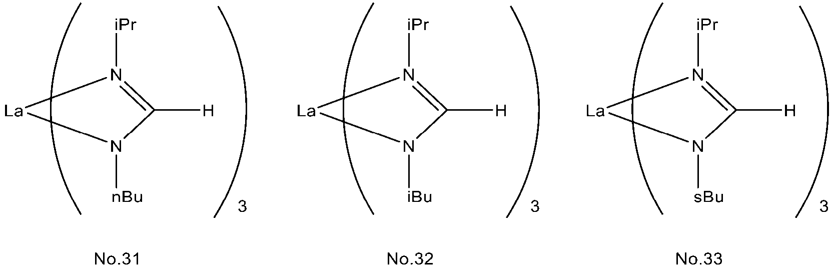

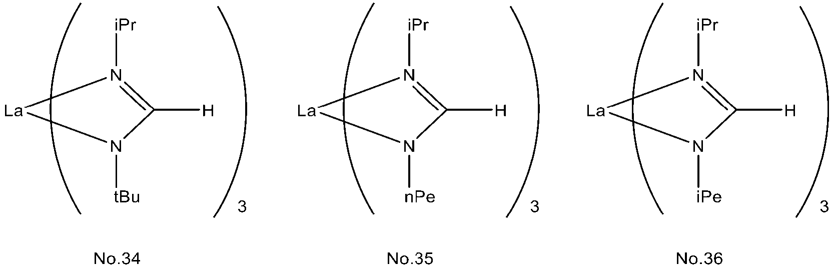

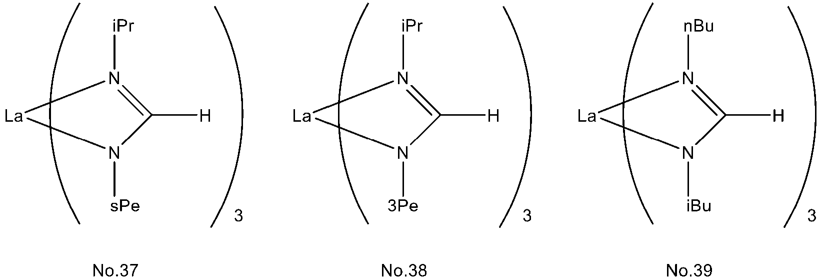

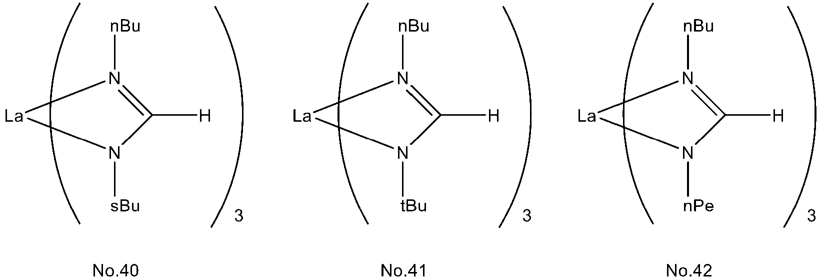

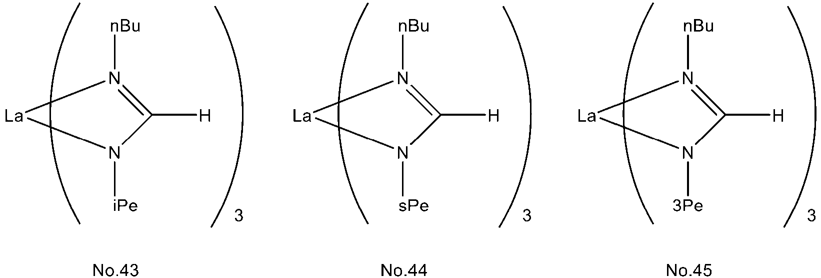

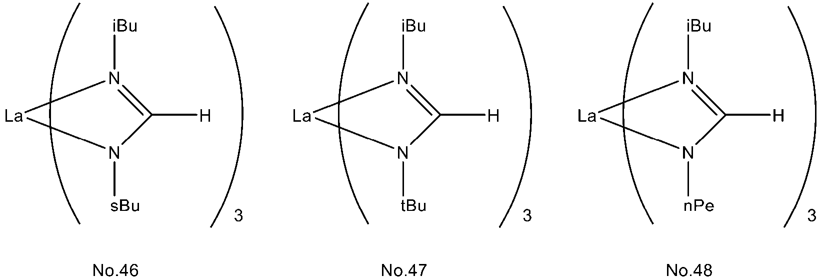

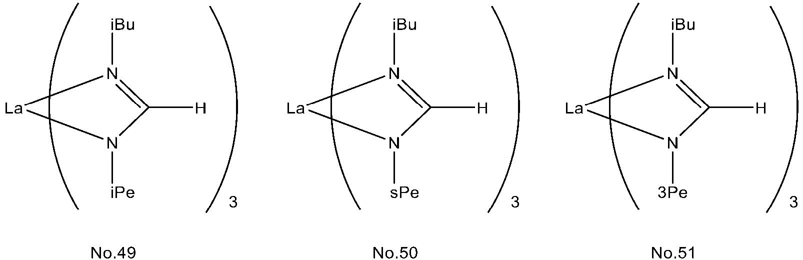

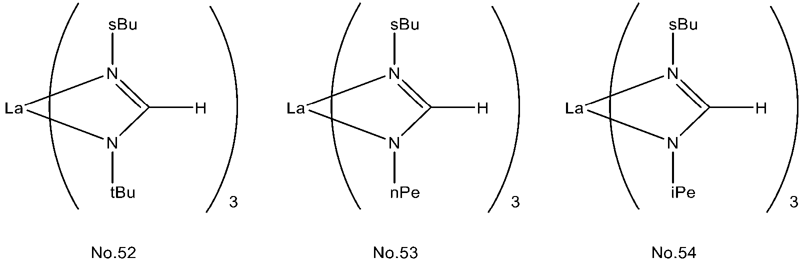

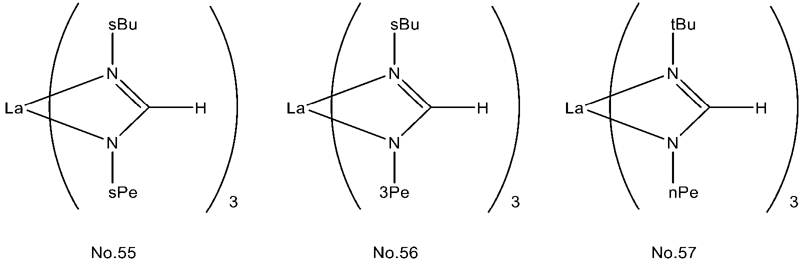

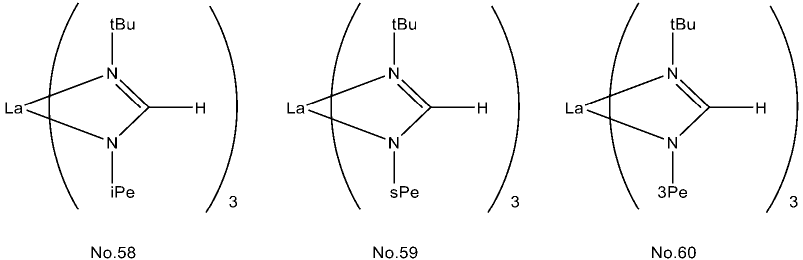

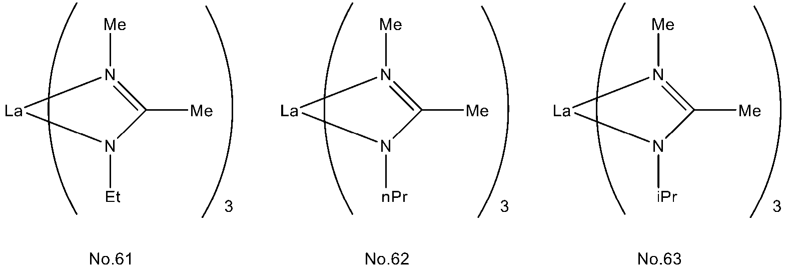

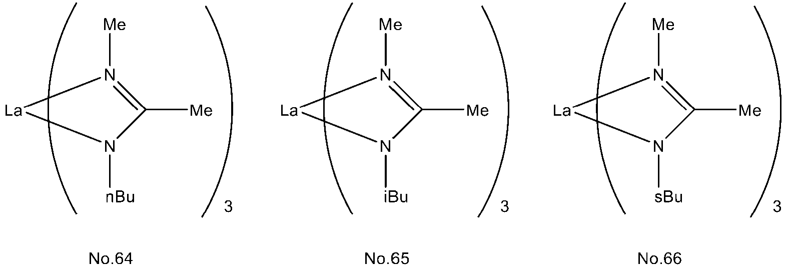

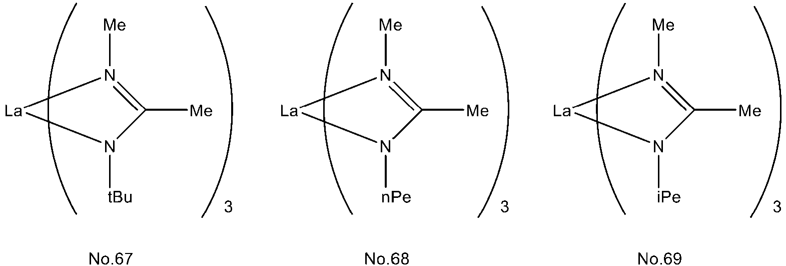

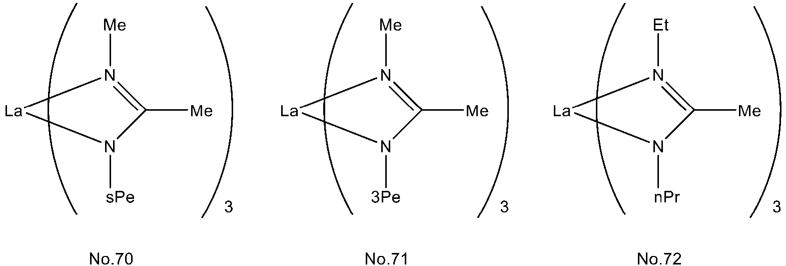

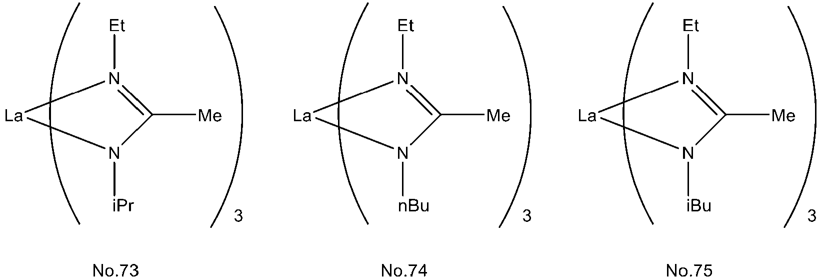

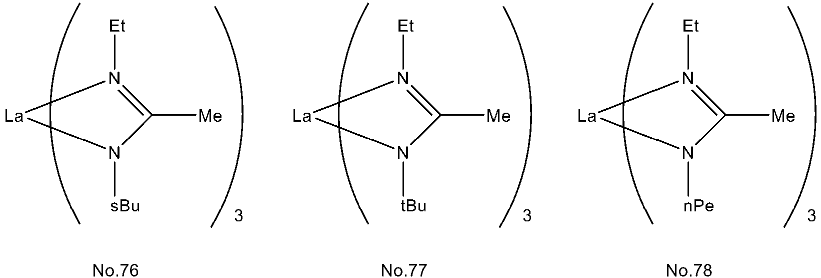

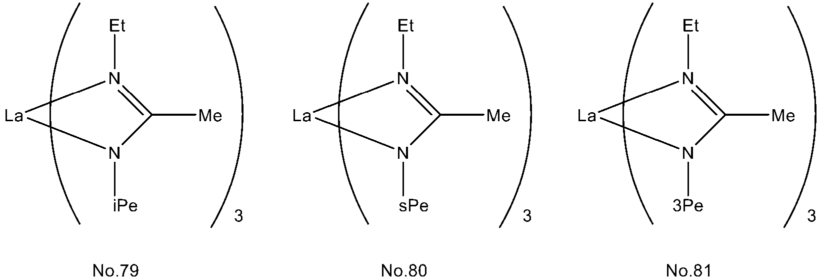

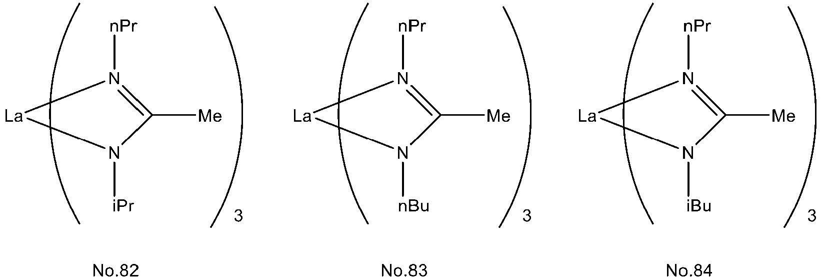

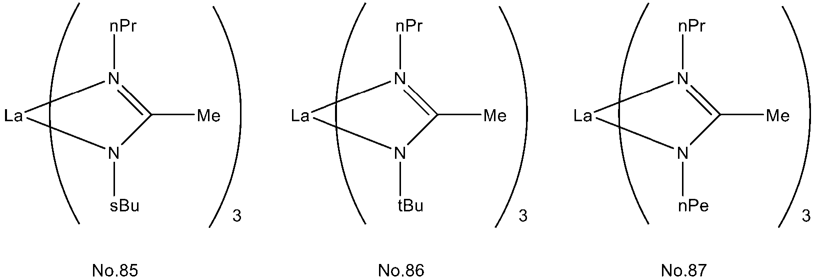

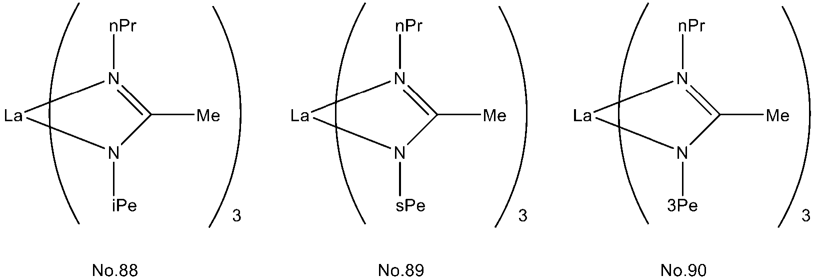

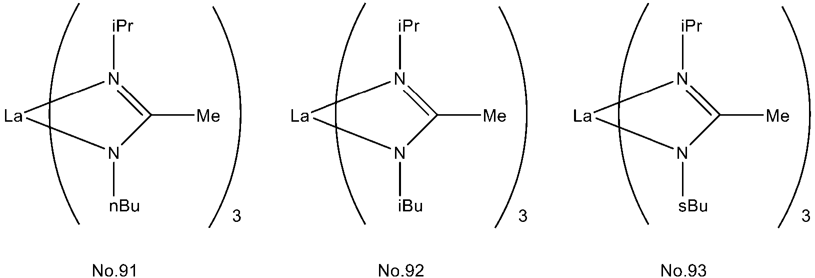

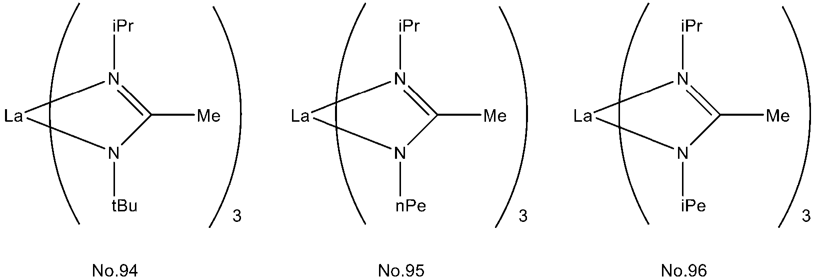

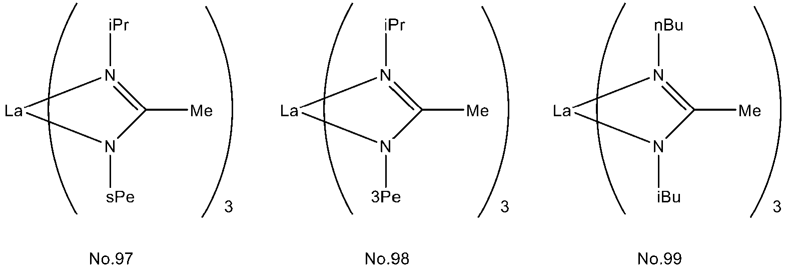

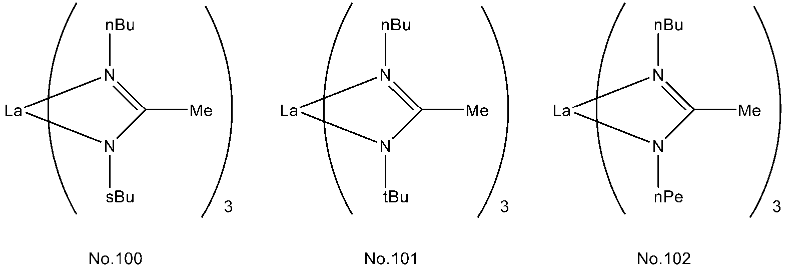

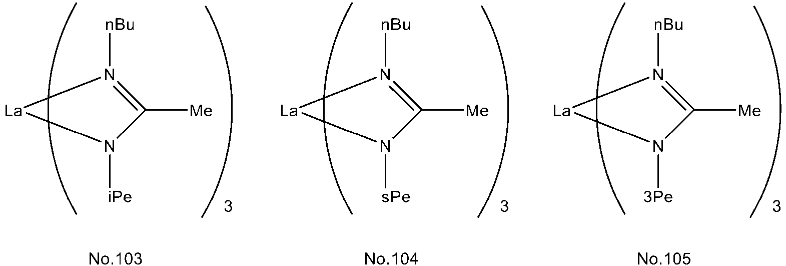

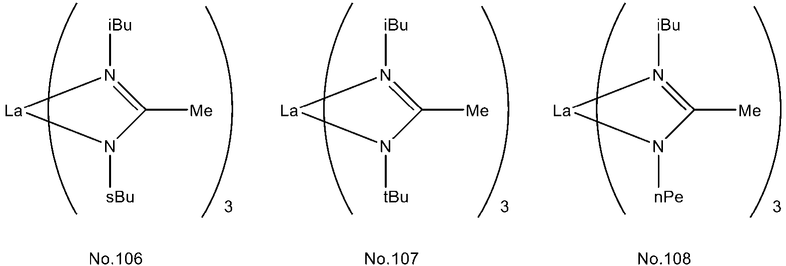

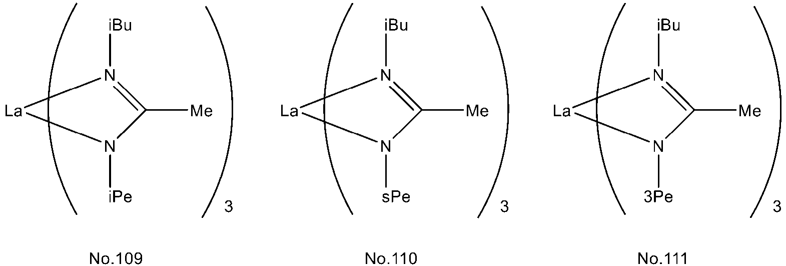

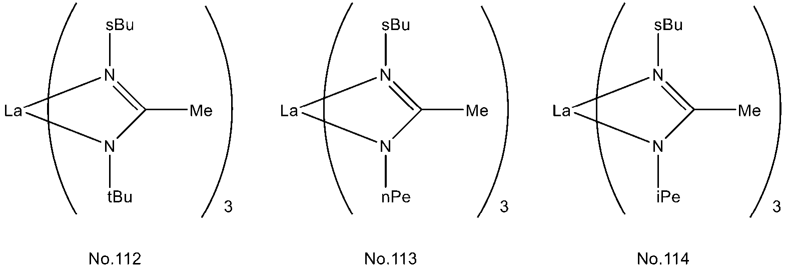

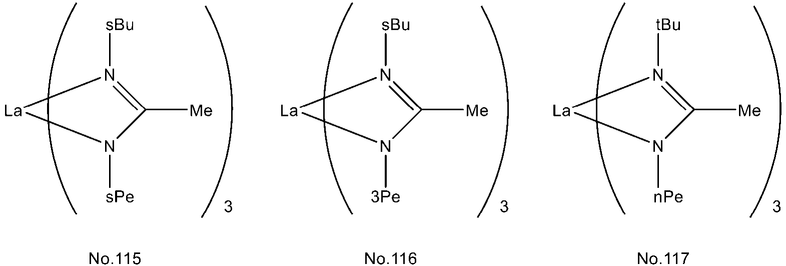

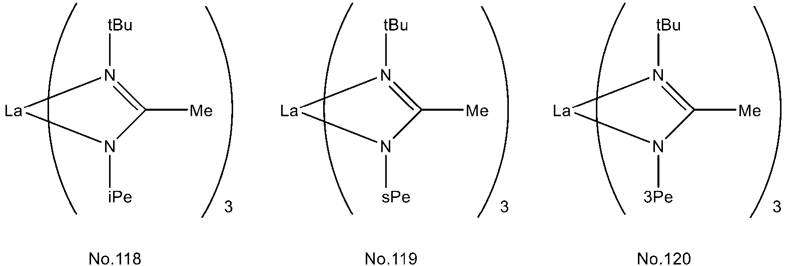

Among the above-mentioned compounds, Compounds No. 33, No. 37, and No. 38 are preferred, Compounds No. 33 and No. 37 are more preferred, and Compound No. 37 is most preferred.

The compound of the present invention is not particularly limited by a production method therefor, and may be produced by applying a well-known synthesis method. For example, the compound represented by the general formula (1) may be obtained by mixing La[N(SiMe₃)₂]₃ and a ligand having a corresponding structure in the presence or absence of a solvent, stirring the mixture to cause a reaction therebetween, and then removing the solvent, followed by distillation.

### [Thin-film Forming Raw Material]

A thin-film forming raw material according to a second aspect of the present invention comprises the above-mentioned compound represented by the general formula (1). The thin-film forming raw material of the present invention only needs to comprise the compound represented by the general formula (1) as a precursor of a thin-film, and the composition thereof varies depending on the kind of the target thin-film. For example, when a thin-film containing only a lanthanum atom as a metal is produced, the thin-film forming raw material is free of a metal compound other than lanthanum and a semimetal compound. Meanwhile, when a thin-film containing a lanthanum atom and a metal other than the lanthanum atom and/or a semimetal is produced, the thin-film forming raw material may comprise, in addition to the compound represented by the general formula (1), a compound containing a desired metal and/or a compound containing a semimetal (hereinafter sometimes referred to as "other precursor").

In the case of a multi-component ALD method in which a plurality of precursors are used, there is no particular limitation on the other precursor that may be used together with the compound represented by the general formula (1), and a well-known general precursor used for the thin-film forming raw material for an ALD method may be used.

Examples of the above-mentioned other precursor include compounds of: one kind or more kinds selected from the group consisting of compounds used as organic ligands, such as an alcohol compound, a glycol compound, a β-diketone compound, a cyclopentadiene compound, and an organic amine compound; and silicon or a metal. In addition, examples of the kind of the metal in the precursor include lithium, sodium, potassium, magnesium, calcium, strontium, barium, titanium, zirconium, hafnium, vanadium, niobium, tantalum, chromium, molybdenum, tungsten, manganese, iron, osmium, ruthenium, cobalt, rhodium, iridium, nickel, palladium, platinum, copper, silver, gold, zinc, aluminum, gallium, indium, germanium, lead, antimony, bismuth, radium, scandium, yttrium, lanthanum, cerium, praseodymium, neodymium, promethium, samarium, europium, gadolinium, terbium, dysprosium, holmium, erbium, thulium, ytterbium, and lutetium.

Examples of the alcohol compound to be used as the organic ligand in the above-mentioned other precursor include: alkyl alcohols, such as methanol, ethanol, propanol, isopropyl alcohol, butanol, sec-butyl alcohol, isobutyl alcohol, tert-butyl alcohol, pentyl alcohol, isopentyl alcohol, and tert-pentyl alcohol; ether alcohols, such as 2-methoxyethanol, 2-ethoxyethanol, 2-butoxyethanol, 2-(2-methoxyethoxy)ethanol, 2-methoxy-1-methylethanol, 2-methoxy-1,1-dimethylethanol, 2-ethoxy-1,1-dimethylethanol, 2-isopropoxy-1,1-dimethylethanol, 2-butoxy-1,1-dimethylethanol, 2-(2-methoxyethoxy)-1,1-dimethylethanol, 2-propoxy-1,1-diethylethanol, 2-sec-butoxy-1,1-diethylethanol, and 3-methoxy-1,1-dimethylpropanol; and dialkylamino alcohols, such as dimethylaminoethanol, ethylmethylaminoethanol, diethylaminoethanol, dimethylamino-2-pentanol, ethylmethylamino-2-pentanol, dimethylamino-2-methyl-2-pentanol, ethylmethylamino-2-methyl-2-pentanol, and diethylamino-2-methyl-2-pentanol.

Examples of the glycol compound to be used as the organic ligand in the above-mentioned other precursor include 1,2-ethanediol, 1,2-propanediol, 1,3-propanediol, 2,4-hexanediol, 2,2-dimethyl-1,3-propanediol, 2,2-diethyl-1,3-propanediol, 1,3-butanediol, 2,4-butanediol, 2,2-diethyl-1,3-butanediol, 2-ethyl-2-butyl-1,3-propanediol, 2,4-pentanediol, 2-methyl-1,3-propanediol, 2-methyl-2,4-pentanediol, 2,4-hexanediol, and 2,4-dimethyl-2,4-pentanediol.

Examples of the β-diketone compound to be used as the organic ligand in the above-mentioned other precursor include: alkyl-substituted β-diketones, such as acetylacetone, hexane-2,4-dione, 5-methylhexane-2,4-dione, heptane-2,4-dione, 2-methylheptane-3,5-dione, 5-methylheptane-2,4-dione, 6-methylheptane-2,4-dione, 2,2-dimethylheptane-3,5-dione, 2,6-dimethylheptane-3,5-dione, 2,2,6-trimethylheptane-3,5-dione, 2,2,6,6-tetramethylheptane-3,5-dione, octane-2,4-dione, 2,2,6-trimethyloctane-3,5-dione, 2,6-dimethyloctane-3,5-dione, 2,9-dimethylnonane-4,6-dione, 2-methyl-6-ethyldecane-3,5-dione, and 2,2-dimethyl-6-ethyldecane-3,5-dione; fluorine-substituted alkyl β-diketones, such as 1,1,1-trifluoropentane-2,4-dione, 1,1,1-trifluoro-5,5-dimethylhexane-2,4-dione, 1,1,1,5,5,5-hexafluoropentane-2,4-dione, and 1,3-diperfluorohexylpropane-1,3-dione; and ether-substituted β-diketones, such as 1,1,5,5-tetramethyl-1-methoxyhexane-2,4-dione, 2,2,6,6-tetramethyl-1-methoxyheptane-3,5-dione, and 2,2,6,6-tetramethyl-1-(2-methoxyethoxy)heptane-3,5-dione.

Examples of the cyclopentadiene compound to be used as the organic ligand in the above-mentioned other precursor include cyclopentadiene, methylcyclopentadiene, ethylcyclopentadiene, propylcyclopentadiene, isopropylcyclopentadiene, butylcyclopentadiene, sec-butylcyclopentadiene, isobutylcyclopentadiene, tert-butylcyclopentadiene, dimethylcyclopentadiene, tetramethylcyclopentadiene, and pentamethylcyclopentadiene.

Examples of the organic amine compound to be used as the organic ligand of the above-mentioned other precursor include methylamine, ethylamine, propylamine, isopropylamine, butylamine, sec-butylamine, tert-butylamine, isobutylamine, dimethylamine, diethylamine, dipropylamine, diisopropylamine, ethylmethylamine, propylmethylamine, and isopropylmethylamine.

The above-mentioned other precursors are known in the art, and production methods therefor are also known. As an example of the production method, for example, when the alcohol compound is used as the organic ligand, the precursor may be produced through a reaction between an inorganic salt of the metal described above or a hydrate thereof and an alkali metal alkoxide of the alcohol compound. In this case, examples of the inorganic salt of the metal or the hydrate thereof may include a halide and a nitrate of the metal. Examples of the alkali metal alkoxide may include a sodium alkoxide, a lithium alkoxide, and a potassium alkoxide.

In such a multi-component ALD method as described above, there are given a method involving vaporizing and supplying the thin-film forming raw material independently for each component (hereinafter sometimes referred to as "single source method"), and a method involving vaporizing and supplying a mixed raw material obtained by mixing a multi-component raw material with desired composition in advance (hereinafter sometimes referred to as "cocktail source method"). In the case of the single source method, the above-mentioned other precursor is preferably a compound that is similar to the compound represented by the general formula (1) in the behavior of thermal decomposition and/or oxidative decomposition. In the case of the cocktail source method, the above-mentioned other precursor is preferably a compound that is similar to the compound represented by the general formula (1) in the behavior of thermal decomposition and/or oxidative decomposition, and in addition, causes no change through a chemical reaction or the like at the time of mixing.

In the case of the cocktail source method out of the multi-component ALD methods, a mixture of the compound represented by the general formula (1) and the other precursor or a mixed solution obtained by dissolving the mixture in an organic solvent may be used as the thin-film forming raw material.

There is no particular limitation on the above-mentioned organic solvent, and a well-known general organic solvent may be used. Examples of the organic solvent include: acetic acid esters, such as ethyl acetate, butyl acetate, and methoxyethyl acetate; ethers, such as tetrahydrofuran, tetrahydropyran, ethylene glycol dimethyl ether, diethylene glycol dimethyl ether, triethylene glycol dimethyl ether, dibutyl ether, and dioxane; ketones, such as methyl butyl ketone, methyl isobutyl ketone, ethyl butyl ketone, dipropyl ketone, diisobutyl ketone, methyl amyl ketone, cyclohexanone, and methylcyclohexanone; hydrocarbons, such as hexane, cyclohexane, methylcyclohexane, dimethylcyclohexane, ethylcyclohexane, heptane, octane, toluene, and xylene; hydrocarbons each having a cyano group, such as 1-cyanopropane, 1-cyanobutane, 1-cyanohexane, cyanocyclohexane, cyanobenzene, 1,3-dicyanopropane, 1,4-dicyanobutane, 1,6-dicyanohexane, 1,4-dicyanocyclohexane, and 1,4-dicyanobenzene; and pyridine and lutidine. Those organic solvents may be used alone or as a mixture thereof depending on the solubility of a solute, a relationship among a usage temperature, a boiling point, and a flash point, and the like.

When the thin-film forming raw material of the present invention is a mixed liquid containing the above-mentioned organic solvent, from the viewpoint that a high-quality lanthanum-containing thin-film having a small residual carbon amount is easily formed, the amount of the entire precursors in the thin-film forming raw material is adjusted to preferably from 0.01 mol/L to 2.0 mol/L, more preferably from 0.05 mol/L to 1.0 mol/L.

Herein, when the thin-film forming raw material is free of any precursor other than the compound represented by the general formula (1), the amount of the entire precursors refers to the amount of the compound represented by the general formula (1). When the thin-film forming raw material comprises any other precursor in addition to the compound represented by the general formula (1), the amount of the entire precursors refers to the total amount of the compound represented by the general formula (1) and the other precursor.

The thin-film forming raw material of the present invention may comprise a nucleophilic reagent as required in order to improve the stability of each of the compound represented by the general formula (1) and the other precursor.

Examples of the nucleophilic reagent include: ethylene glycol ethers, such as glyme, diglyme, triglyme, and tetraglyme; crown ethers, such as 18-crown-6, dicyclohexyl-18-crown-6, 24-crown-8, dicyclohexyl-24-crown-8, and dibenzo-24-crown-8; polyamines, such as ethylenediamine, N,N'-tetramethylethylenediamine, diethylenetriamine, triethylenetetramine, tetraethylenepentamine, pentaethylenehexamine, 1,1,4,7,7-pentamethyldiethylenetriamine, 1,1,4,7,10,10-hexamethyltriethylenetetramine, and triethoxytriethyleneamine; cyclic polyamines, such as cyclam and cyclen; heterocyclic compounds, such as pyridine, pyrrolidine, piperidine, morpholine, N-methylpyrrolidine, N-methylpiperidine, N-methylmorpholine, tetrahydrofuran, tetrahydropyran, 1,4-dioxane, oxazole, thiazole, and oxathiolane; β-keto esters, such as methyl acetoacetate, ethyl acetoacetate, and 2-methoxyethyl acetoacetate; and β-diketones, such as acetylacetone, 2,4-hexanedione, 2,4-heptanedione, 3,5-heptanedione, and dipivaloylmethane. From the viewpoint that the stability is easily controlled, the usage amount of each of those nucleophilic reagents falls within the range of preferably from 0.1 mol to 10 mol, more preferably from 1 mol to 4 mol with respect to 1 mol of the amount of the entire precursors.

It is desired that the thin-film forming raw material of the present invention be prevented from containing impurity metal elements other than the components for forming the raw material, impurity halogens such as impurity chlorine, and impurity organic substances to the extent possible. The content of each of the impurity metal elements is preferably 100 ppb or less, more preferably 10 ppb or less, and the total content thereof is preferably 1 ppm or less, more preferably 100 ppb or less. In particular, when the raw material is used as a gate insulating film, a gate film, a barrier layer, or a wiring layer of an LSI, it is required to reduce the contents of alkali metal elements and alkaline-earth metal elements that influence the electrical characteristics of a thin-film to be obtained. The content of the impurity halogens is preferably 100 ppm or less, more preferably 10 ppm or less, still more preferably 1 ppm or less. The total content of the impurity organic substances is preferably 500 ppm or less, more preferably 50 ppm or less, still more preferably 10 ppm or less. In addition, moisture causes generation of particles in the thin-film forming raw material and generation of particles during thin-film formation, and hence it is better to remove moisture from the precursor, the organic solvent, and the nucleophilic reagent as much as possible in advance at the time of use in order to reduce moisture in each of the precursor, the organic solvent, and the nucleophilic reagent. The moisture content of each of the precursor, the organic solvent, and the nucleophilic reagent is preferably 10 ppm or less, more preferably 1 ppm or less. When the contents of the impurity metal elements, the impurity halogens, and the impurity organic substances, and the moisture content in the thin-film forming raw material of the present invention are equal to or lower than the above-mentioned numerical values, a high-quality lanthanum-containing thin-film having a small residual carbon amount is easily formed.

It is preferred that the thin-film forming raw material of the present invention be prevented from containing particles to the extent possible in order to reduce or prevent particle contamination of a thin-film to be formed. Specifically, from the viewpoint that a uniform lanthanum-containing thin-film is easily obtained, in particle measurement with a light scattering liquid particle detector in a liquid phase, it is preferred that the number of particles larger than 0.3 µm be 100 or less in 1 ml of the thin-film forming raw material, and it is preferred that the number of particles larger than 0.2 µm be 100 or less in 1 mL of the thin-film forming raw material.

### [Method of producing Thin-film]

Next, a method of producing a thin-film according to a fourth aspect of the present invention is described.

The method of producing a thin-film of the present invention comprises forming a lanthanum-containing thin-film on a surface of a substrate through use of the above-mentioned thin-film forming raw material.

It is preferred that the method of producing a thin-film of the present invention comprise: a raw material introduction step of introducing a raw material gas obtained by vaporizing the above-mentioned thin-film forming raw material into a film formation chamber (100) having a substrate set therein; and a thin-film formation step of subjecting the compound represented by the general formula (1) to heating and/or plasmatization to form the lanthanum-containing thin-film on the surface of the substrate. In particular, from the viewpoint that a high-quality lanthanum-containing thin-film having a small residual carbon amount is easily obtained, it is preferred that the method of producing a thin-film of the present invention further comprise, between the raw material introduction step and the thin-film formation step, a precursor thin-film formation step of forming a precursor thin-film on the surface of the substrate through use of the thin-film forming raw material, and that the thin-film formation step be a step of subjecting the compound represented by the general formula (1) to heating and/or plasmatization in the presence of a reactive gas.

There are no particular limitations on a transportation and supply method for the raw material, production conditions, a production apparatus, and the like, and well-known general conditions and methods may be used.

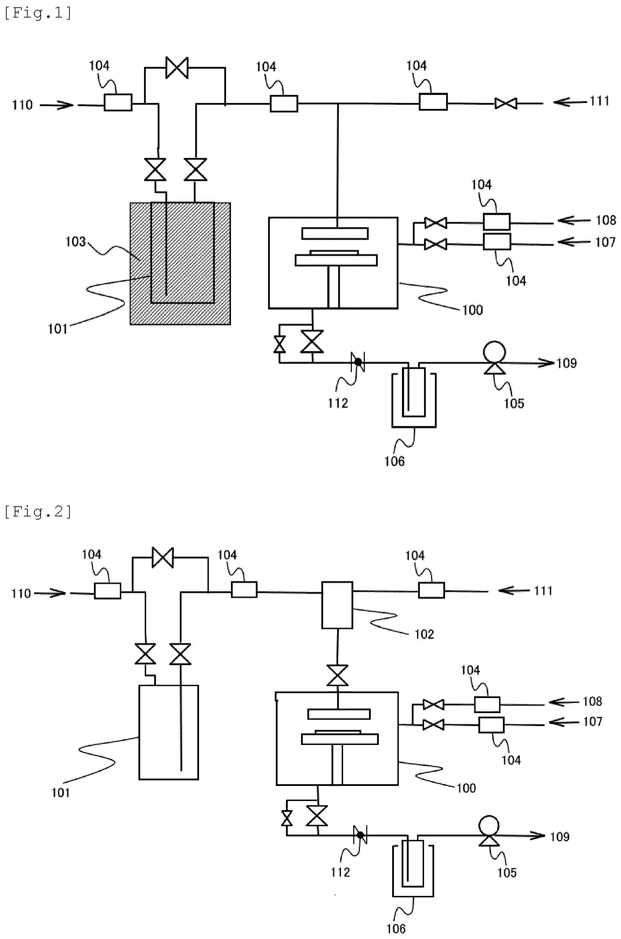

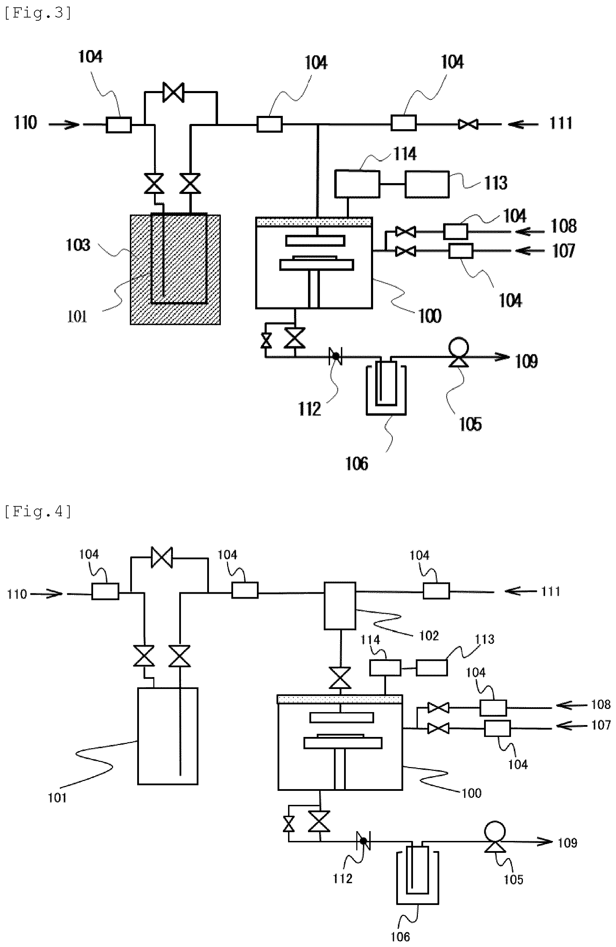

A well-known ALD apparatus may be used as an apparatus for producing a thin-film through use of the thin-film forming raw material of the present invention. Specific examples of the apparatus include: such an apparatus capable of performing bubbling supply of a precursor as illustrated in each of FIG. 1 and FIG. 3; and such an apparatus including a vaporization chamber (102) as illustrated in each of FIG. 2 and FIG. 4. Another specific example thereof is such an apparatus capable of subjecting the reactive gas to plasma treatment as illustrated in each of FIG. 3 and FIG. 4. The apparatus is not limited to such single-substrate type apparatus each including a film formation chamber (100) as illustrated in FIG. 1 to FIG. 4, and an apparatus capable of simultaneously processing a large number of substrates through use of a batch furnace may also be used. Those apparatus may also be used as CVD apparatus.

The steps of such method of producing a thin-film are described below.

The thin-film forming raw material to be used in the method of producing a thin-film of the present invention is the same as that described in the above-mentioned [Thin-film Forming Raw Material] section, and hence the description thereof is omitted.

The raw material introduction step in the present invention is a step of introducing a raw material gas obtained by vaporizing the thin-film forming raw material into a film formation chamber (100) having the substrate set therein.

As a method of introducing the raw material gas obtained by vaporizing the thin-film forming raw material into the film formation chamber having the substrate set therein, there are given, for example, a gas transportation method, a liquid transportation method, a single source method, and a cocktail source method.

An example of the gas transportation method is such a method as illustrated in each of FIG. 1 and FIG. 3 involving heating and/or vaporizing the thin-film forming raw material in a raw material container (101) having stored therein the thin-film forming raw material, to thereby provide the raw material gas, and introducing the raw material gas into the film formation chamber (100) having the substrate set therein together with a carrier gas, such as argon, nitrogen, or helium, as required.

An example of the liquid transportation method is such a method as illustrated in each of FIG. 2 and FIG. 4 involving transporting the thin-film forming raw material to a vaporization chamber (102) under a state of a liquid or a solution, heating and/or vaporizing the thin-film forming raw material in the vaporization chamber (102), to thereby provide the raw material gas, and introducing the raw material gas into the film formation chamber (100).

The single source method is a transportation and supply method for the thin-film forming raw material comprising multi-component precursors, and an example thereof is a method involving vaporizing and supplying the precursors independently of each other.

An example of the cocktail source method is a method involving vaporizing and supplying a mixed raw material obtained by mixing multi-component precursors with desired composition in advance. The thin-film forming raw material comprising the multi-component precursors may comprise the above-mentioned nucleophilic reagent or the like.

The step of vaporizing the thin-film forming raw material of the present invention to provide the raw material gas may be performed in the raw material container or in the vaporization chamber as described above. In any case, from the viewpoint that a high-quality thin-film is easily formed, it is preferred that the thin-film forming raw material be vaporized at from 0°C to 200°C.

In addition, when the thin-film forming raw material is vaporized in the raw material container (101) or in the vaporization chamber (102) to provide the raw material gas, from the viewpoint that the vaporization of the thin-film forming raw material becomes satisfactory, a pressure in the raw material container and a pressure in the vaporization chamber each fall within the range of preferably from 1 Pa to 10,000 Pa.

As a material for the substrate to be set in the film formation chamber (100), there are given, for example: silicon; ceramics, such as silicon nitride, titanium nitride, tantalum nitride, titanium oxide, molybdenum oxide, zirconium oxide, hafnium oxide, and lanthanum oxide; glass; and metals, such as metal cobalt and metal molybdenum. The shape of the substrate is, for example, a plate shape, a spherical shape, a fibrous shape, or a scaly shape. The surface of the substrate may be planar, or may have a three-dimensional structure such as a trench structure.

The thin-film formation step is a step of forming a lanthanum-containing thin-film on the surface of the substrate by subjecting the compound represented by the general formula (1) to heating and/or plasmatization ("heating and/or plasmatization" is hereafter sometimes referred to as "heating and the like").

The thin-film formation step is performed in the film formation chamber (100) having the substrate set therein as illustrated in FIG. 1 to FIG. 4. In the thin-film formation step, the compound in the raw material gas introduced into the film formation chamber (100) is decomposed by heating and the like, and a lanthanum-containing thin-film is formed by depositing metal lanthanum or a compound containing lanthanum on the surface of the substrate. In each of FIG. 1 to FIG. 4, there is illustrated an example in which the compound in the raw material gas introduced into the film formation chamber (100) is heated in the presence of the reactive gas. In this example, the lanthanum-containing thin-film is formed by the reaction between the compound and the reactive gas.

The heating temperature of the compound in the thin-film formation step only needs to fall within the range of from room temperature to 700°C, but from the viewpoint that a high-quality lanthanum-containing thin-film is easily formed, the heating temperature falls within the range of preferably from 150°C to 600°C.

In the thin-film formation step, the condition under which the compound is subjected to plasmatization by the application of a voltage is preferably a power of from 10 W to 1,500 W, more preferably a power of from 50 W to 600 W because significant damage to the substrate occurs when the power is too large.

When the method further comprises, between the raw material introduction step and the thin-film formation step, a precursor thin-film formation step described later, from the viewpoint that a high-quality lanthanum-containing thin-film having a small residual carbon amount is easily obtained, the compound is preferably subjected to heating and/or plasmatization in the presence of the reactive gas, and the compound is more preferably heated in the presence of the reactive gas. Examples of the reactive gas include: oxidizing gases, such as oxygen, ozone, nitrogen dioxide, nitrogen monoxide, water vapor, hydrogen peroxide, formic acid, acetic acid, and acetic anhydride; reducing gases such as hydrogen; and nitriding gases, such as an organic amine compound, for example, a monoalkylamine, a dialkylamine, a trialkylamine, or an alkylene diamine, hydrazine, and ammonia. Those reactive gases may be used alone or as a mixture thereof. A reactive gas containing at least one kind selected from the group consisting of: hydrogen; ammonia; oxygen; ozone; and water vapor out of those reactive gases is preferably used from the viewpoint that a high-quality lanthanum-containing thin-film having a small residual carbon amount is easily formed. The compound represented by the general formula (1) has a property of satisfactorily reacting with an oxidizing gas, and hence an oxidizing gas such as water vapor is more preferably used as the reactive gas.

The method of producing a thin-film of the present invention preferably further comprises, between the raw material introduction step and the thin-film formation step, the precursor thin-film formation step of forming a precursor thin-film on the surface of the substrate through use of the thin-film forming raw material. The precursor thin-film only needs to be a precursor thin-film capable of forming the lanthanum-containing thin-film in the thin-film formation step.

Such precursor thin-film may be formed by depositing (adsorbing) the compound represented by the general formula (1) in the raw material gas introduced into the film formation chamber (100) having the substrate set therein onto the surface of the substrate. In this case, the substrate may be heated, or the inside of the film formation chamber (100) may be heated.

The conditions under which the precursor thin-film is formed are not particularly limited, and for example, a reaction temperature (temperature of the substrate when the compound is deposited on the surface of the substrate), a reaction pressure (pressure in the film formation chamber when the compound is deposited on the surface of the substrate), a deposition rate, and the like may be appropriately determined depending on the kind of the thin-film forming raw material. From the viewpoint that a precursor thin-film is easily formed uniformly, the reaction temperature falls within the range of preferably from 0°C to 700°C, more preferably from 150°C to 600°C.

The method of producing a thin-film of the present invention preferably further comprises, after the formation of the precursor thin-film in the precursor thin-film formation step or after the formation of the lanthanum-containing thin-film in the thin-film formation step, an evacuation step of evacuating the raw material gas, the unreacted reactive gas, and a by-product gas from the film formation chamber (100). In this case, it is ideal that the raw material gas that has not deposited, the reactive gas, and the by-product gas be completely evacuated from the film formation chamber, but it is not always required that the gases be completely evacuated. As an evacuation method, there are given, for example: a method involving purging a system of the film formation chamber with an inert gas, such as helium, nitrogen, or argon; a method involving performing evacuation by decompressing the system of the film formation chamber; and a combination of these methods. The degree of decompression in the case of decompressing the system preferably falls within the range of from 0.01 Pa to 300 Pa, and from the viewpoint that the evacuation of the raw material gas, the unreacted reactive gas, and the by-product gas is promoted, the degree of decompression more preferably falls within the range of from 0.01 Pa to 100 Pa.

In order to achieve more satisfactory electrical characteristics of the lanthanum-containing thin-film, the method of producing a thin-film of the present invention may further comprise an annealing step after the formation of the thin-film. In the annealing step, annealing treatment may be performed under an inert atmosphere, an oxidizing atmosphere, or a reducing atmosphere, and in the case where step embedding is required, a reflow step may be provided. From the viewpoint that a high-quality lanthanum-containing thin-film having a small residual carbon amount is easily produced, a temperature in the annealing step falls within the range of preferably from 250°C to 500°C.

The method of producing a thin-film of the present invention may be a method involving performing the thin-film formation step only once, or may be a method involving performing the thin-film formation step twice or more, but is preferably a method involving performing the thin-film formation step twice or more. In the present invention, the formation of a lanthanum-containing thin-film having a desired thickness only needs to be performed as follows: the raw material gas introduction step, the precursor thin-film formation step, the evacuation step, the thin-film formation step, and the evacuation step are sequentially performed, and the formation of the lanthanum-containing thin-film through the series of operations is defined as one cycle; and the cycle is repeated a plurality of times until a lanthanum-containing thin-film having a required thickness is obtained. The thickness of the lanthanum-containing thin-film to be formed may be controlled by the number of cycles. From the viewpoint that a uniform lanthanum-containing thin-film is easily obtained, the deposition rate of the lanthanum-containing thin-film obtained per cycle falls within the range of preferably from 0.001 nm/min. to 100 nm/min., more preferably from 0.005 nm/min. to 50 nm/min.

### [Thin-film formed from Thin-film Forming Raw Material]

A thin-film formed from the thin-film forming raw material according to a third aspect of the present invention is, for example, a thin-film of metal lanthanum, a lanthanum oxide, or a lanthanum nitride, and a thin-film of a lanthanum oxide can be efficiently formed by the above-mentioned method of producing a thin-film. The thin-film of the present invention can be obtained as a desired kind of thin-film by appropriately selecting the other precursor, the reactive gas, and the production conditions in the method of producing a thin-film described above. The thin-film of the present invention is excellent in electrical characteristics and optical characteristics, and hence can be widely used in the production of, for example, electrode materials of memory devices typified by DRAM devices, wiring materials to be used in semiconductor devices such as a logic device, diamagnetic films used for recording layers of hard disks, and catalyst materials for polymer electrolyte fuel cells.

In addition, the carbon content in the thin-film is preferably 1 atm% or less, more preferably 0.5 atm% or less, still more preferably 0.1 atm% or less.

### Examples

The present invention is described in more detail below by way of the Examples. However, the present invention is not limited by the following Examples and the like.

### [Example 1-1] Production of Precursor of Ligand of Compound No. 33

Isopropyl isocyanate was added dropwise to a solution of 57.4 g of sec-butylamine (0.78 mol) and 800 mL of THF under cooling with ice water, followed by stirring at room temperature overnight. The solvent was removed, and dichloromethane (1 L) and 270 g (2.67 mol) of triethylamine were added to the residue. A solution of 299 g (1.57 mol) of p-toluenesulfonyl chloride and 300 mL of dichloromethane cooled with ice water was added dropwise to the mixture, followed by stirring under reflux for 3 hours. 760 g of a 40% potassium carbonate aqueous solution was added to the resultant under cooling with ice water to separate the resultant into oil and water layers, and the water layer was extracted with dichloromethane. The organic layers were combined and washed with water. After the solvent was removed, diethyl ether was added to the residue, and the solution was taken out by decantation. After the solvent was removed, the residue was distilled to provide 80.7 g (yield: 73%) of an intended product.

### (Analysis Values)

(1) ¹H-NMR (solvent: chloroform-d) (chemical shift: multiplicity: number of H atoms)
(0.947: triplet: 3) (1.217: doublet: 3) (1.225: doublet: 6) (1.456-1.530: multiplet: 2) (3.320: sextet: 1) (3.559: septet: 1)

### [Example 1-2] Production of Ligand of Compound No. 33

A hexane solution of 46.3 g (0.33 mol) of diisobutylaluminum hydride (DIBAL) cooled with ice water was added dropwise to a solution of 43.5 g (0.31 mol) of the precursor obtained in Example 1-1 and 500 mL of THF, followed by stirring at room temperature overnight. After 228 mL of MeOH cooled with ice water was added dropwise thereto, the mixture was washed with a 30% Rochelle salt aqueous solution, and the organic layer was dried with sodium sulfate. After the solvent was removed, the residue was distilled to provide 33.9 g (yield: 77%) of an intended product.

### (Analysis Values)

(1) ¹H-NMR (solvent: chloroform-d) (chemical shift: multiplicity: number of H atoms)
(0.854: triplet: 3) (1.113: doublet: 3) (1.130: doublet: 6) (1.352-1.506: multiplet: 2) (2.914-3.234: multiplet: 1) (3.323-3.603: multiplet: 1) (3.815: broad: 1) (7.354: singlet: 1)

### [Example 1-3] Production of Compound No. 33

Under an argon atmosphere, La[N(SiMe₃)₂]₃ (2.21 g, 3.56 mmol) and toluene (5.2 mL) were loaded into a 100 mL three-necked flask, and the ligand (1.61 g, 7.12 mmol) obtained in Example 1-2 was added dropwise thereto at room temperature. The mixture was stirred at room temperature for 24 hours, and then the solvent was removed. The residue was distilled at a temperature of 160°C and a pressure of 35 Pa with a Kugelrohr distillation apparatus to provide an intended product in a yield of 0.40 g and a percent yield of 20%.

### (Analysis Values)

### (1) Normal-pressure TG-DTA

50% mass loss temperature: 252°C (Ar flow rate: 100 ml/min., temperature increase at 10°C/min., sample amount: 10.294 mg)

### (2) Reduced-pressure TG-DTA

50% mass loss temperature: 160°C (10 Torr, Ar flow rate: 50 ml/min., temperature increase at 10°C/min., sample amount: 9.620 mg)

### (3) ¹H-NMR (solvent: deuterated benzene) (chemical shift: multiplicity: number of H atoms)

(0.918-0.955: triplet: 3)(1.205-1.239: multiplet: 9)(1.468-1.522: quintet: 2)(2.785-20801: multiplet: 1)(3.121-3.152: quintet: 1)(8.380: singlet: 1)

### [Example 2-1] Production of Precursor of Ligand of Compound No. 37

Under an argon atmosphere, isopropyl isothiocyanate (13.2 g, 0.13 mol) and diethyl ether (406 mL) were loaded into a 1 L four-necked flask and cooled with ice. 2-Aminopentane (11.3 g, 0.13 mol) was added dropwise thereto. After the temperature was increased to room temperature, the mixture was stirred for 18 hours. After the solvent was removed, dichloromethane (307 mL) and triethylamine (46.0 g, 0.46 mol) were added to the residue, and the mixture was cooled to -35°C. A solution in which N-bromosuccinimide (24.3 g, 0.14 mol) was suspended in dichloromethane (183 mL) was slowly added thereto. After the temperature was increased to room temperature, the mixture was stirred for 20 hours. After that, a potassium carbonate aqueous solution was added thereto. After the resultant was separated into two layers, the organic layer was recovered. The organic layer was dried with sodium sulfate, followed by filtration and removal of the solvent. The resultant residue was distilled at a bath temperature of 85°C and a pressure of 920 Pa to provide a pale yellow transparent liquid that was an intended product in a yield of 12.7 g and a percent yield of 63%.

### (Analysis Values)

(1) ¹H-NMR (solvent: deuterated benzene) (chemical shift: multiplicity: number of H atoms)
(0.789-0.824: triplet: 3) (1.049-1.065: doublet: 6) (1.073-1.089: doublet: 3) (1.205-1.301: multiplet: 2) (1.347-1.421: multiplet: 2) (3.210-3.257: multiplet: 1) (3.306-3.402: septet: 1)

### [Example 2-2] Production of Ligand of Compound No. 37

Under an argon atmosphere, the precursor (9.87 g, 63.6 mmol) obtained in Example 2-1 and THF (51.4 mL) were loaded into a 300 mL three-necked flask and cooled with ice. A 1 M diisobutylaluminum hydride hexane solution (63.4 mL, 63.4 mmol) was added dropwise thereto. After the temperature was increased to room temperature, the mixture was stirred for 22 hours. After the mixture was quenched with a saturated Rochelle salt aqueous solution, diethyl ether was added thereto to extract an intended product into the organic layer. The organic layer was dried with sodium sulfate, followed by filtration and removal of the solvent. The resultant residue was distilled at a bath temperature of 87°C and a pressure of 540 Pa to provide a colorless transparent liquid that was an intended product in a yield of 7.15 g and a percent yield of 72%.

### [Example 2-3] Production of Compound No. 37

Under an argon atmosphere, La[N(SiMe₃)₂]₃ (30.8 g, 0.0496 mol) and toluene (158 mL) were loaded into a 300 mL three-necked flask and cooled with ice. The ligand (23.3 g, 0.1488 mol) obtained in Example 2-2 was added dropwise thereto. After the temperature was increased to room temperature, the mixture was stirred for 21 hours, and then the solvent was removed. The residue was distilled at a bath temperature of 167°C and a pressure of 79 Pa to provide an intended product in a yield of 21.8 g and a percent yield of 73%.

### (Analysis Values)

### (1) Normal-pressure TG-DTA

50% mass loss temperature: 268°C (Ar flow rate: 100 ml/min., temperature increase at 10°C/min., sample amount: 9.854 mg)

### (2) Reduced-pressure TG-DTA

50% mass loss temperature: 174°C (10 Torr, Ar flow rate: 50 ml/min., temperature increase at 10°C/min., sample amount: 10.006 mg)

### (3) ¹H-NMR (solvent: deuterated benzene) (chemical shift: multiplicity: number of H atoms)

(0.947-0.983: triplet: 9) (1.206-1.242: multiplet: 27) (1.263-1.361: multiplet: 3) (1.417-1.554: multiplet: 9) (2.909-2.922: multiplet: 3) (3.104-3.199: septet: 3) (8.367: singlet: 3)

### [Example 3-1] Production of Precursor of Ligand of Compound No. 38

Under an argon atmosphere, isopropyl isothiocyanate (13.2 g, 0.13 mol) and diethyl ether (406 mL) were loaded into a 1 L three-necked flask and cooled with ice. 3-Aminopentane (11.3 g, 0.13 mol) dissolved in diethyl ether (203 mL) was added dropwise thereto. After the temperature was increased to room temperature, the mixture was stirred for 17 hours. After the solvent was removed, dichloromethane (307 mL) and triethylamine (46.0 g, 0.46 mol) were added to the residue and the mixture was cooled to -55°C. A solution in which N-bromosuccinimide (24.3 g, 0.14 mol) was suspended in dichloromethane (183 mL) was slowly added thereto. After the temperature was increased to room temperature, the mixture was stirred for 23 hours. After that, a potassium carbonate aqueous solution was added thereto. After the resultant was separated into two layers, the organic layer was recovered. The organic layer was dried with sodium sulfate, followed by filtration and removal of the solvent. The resultant residue was distilled at a bath temperature of 85°C and a pressure of 650 Pa to provide an intended product in a yield of 6.52 g and a percent yield of 33%.

### (Analysis Values)

(1) ¹H-NMR (solvent: deuterated benzene) (chemical shift: multiplicity: number of H atoms)
(0.869-0.906: triplet: 6) (1.052-1.068: doublet: 6) (1.324-1.397: multiplet: 4) (2.916-2.980: quintet: 1) (3.334-3.398: septet: 1)

### [Example 3-2] Production of Ligand of Compound No. 38

Under an argon atmosphere, the precursor (6.05 g, 39.2 mmol) obtained in Example 3-1 and THF (31.8 mL) were loaded into a 200 mL four-necked flask and cooled with ice. A 1.02 M diisobutylaluminum hydride hexane solution (38.5 mL, 39.2 mmol) was added dropwise thereto. After the temperature was increased to room temperature, the mixture was stirred overnight. After the mixture was quenched with a saturated Rochelle salt aqueous solution, diethyl ether was added thereto to extract an intended product into the organic layer. The organic layer was dried with sodium sulfate, followed by filtration and removal of the solvent. The resultant residue was distilled at a bath temperature of 85°C and a pressure of 370 Pa to provide an intended product in a yield of 2.39 g and a percent yield of 39%.

### [Example 3-3] Production of Compound No. 38

Under an argon atmosphere, La[N(SiMe₃)₂]₃ (2.00 g, 3.23 mmol) and toluene (10.3 mL) were loaded into a 200 mL four-necked flask and cooled with ice. The ligand (1.51 g, 9.68 mmol) obtained in Example 3-2 was added dropwise thereto. After the temperature was increased to room temperature, the mixture was stirred overnight, and then the solvent was removed. The residue was distilled at a bath temperature of 152°C and a pressure of 27 Pa to provide an intended product in a yield of 0.95 g and a percent yield of 49%.

### (Analysis Values)

### (1) Normal-pressure TG-DTA

50% mass loss temperature: 260°C (Ar flow rate: 100 ml/min., temperature increase at 10°C/min., sample amount: 10.112 mg)

### (2) Reduced-pressure TG-DTA

50% mass loss temperature: 186°C (10 Torr, Ar flow rate: 50 ml/min., temperature increase at 10°C/min., sample amount: 9.690 mg)

### (3) ¹H-NMR (solvent: deuterated benzene) (chemical shift: multiplicity: number of H atoms)

(0.945-0.982: triplet: 18) (1.220-1.235: doublet: 18) (1.460-1.612: multiplet: 12) (2.480: broad: 3) (3.157: broad: 3) (8.384: singlet: 3)

### [Evaluation Example] Evaluation of Physical Properties of Compound

The following evaluations were performed on the compounds obtained in Examples 1 to 3 and Comparative Compound 1 below. The symbol "sBu" in Comparative Compound 1 below represents a sec-butyl group.

### (1) Temperature (°C) at Time of 50 Mass% Loss in Normal-pressure TG-DTA

A change in weight of each of test compounds was measured with a TG-DTA at 760 Torr, an Ar flow rate of 50 mL/min., and a temperature increase rate of 10°C/min. in the scanning temperature range of from 30°C to 600°C, and a temperature (°C) when the weight of the test compound reduced by 50 mass% was evaluated as a "temperature (°C) at the time of a 50 mass% loss in normal-pressure TG-DTA." A lower temperature (°C) at the time of a 50 mass% loss in normal-pressure TG-DTA means that vapor is obtained at a lower temperature. The results are shown in Table 1 below.

### (2) Evaluation of State and Melting Point

The state of each of the compounds at normal pressure and 25°C was visually observed. A compound that was a solid at 25°C was measured for a melting point with a micro melting point-measuring device. It can be determined that a compound having a low melting point is excellent in transportability and is preferred as a thin-film forming raw material. The results are shown in Table 1.

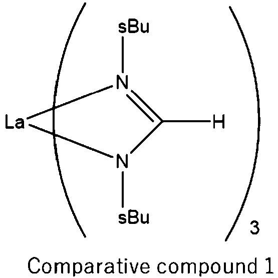

**Table 1**

| | Compound | Temperature at time of 50 mass% loss in normal-pressure TG-DTA [°C] | State at 25°C | Melting point [°C] |
|---|---|---|---|---|
| Evaluation Example 1 | Compound No. 33 | 250 | Solid | 150 |
| Evaluation Example 2 | Compound No. 37 | 265 | Solid | 55 |
| Evaluation Example 3 | Compound No. 38 | 260 | Solid | 200 |
| Comparative Evaluation Example 1 | Comparative Compound 1 | 270 | Solid | 170 |

As shown in Table 1 above, while Comparative Compound 1 has a temperature at the time of a 50 mass% loss in normal-pressure TG-DTA of 270°C, each of the compounds obtained in Examples 1 to 3 has a temperature at the time of a 50 mass% loss in normal-pressure TG-DTA of 265°C or less. Thus, it was understood that the compounds obtained in Examples 1 to 3 were compounds each having a high vapor pressure. In addition, while Comparative Compound 1 has a melting point of 170°C, each of the compounds obtained in Examples 1 and 2 has a melting point of 150°C or less. Thus, it was understood that the compounds obtained in Examples 1 and 2 were compounds excellent in transportability. In particular, the compound obtained in Example 2 has a melting point of 55°C, and thus it was understood that the compound obtained in Example 2 was particularly excellent in transportability.

### <Production of Thin-film by ALD method>

A lanthanum oxide thin-film was produced on a silicon substrate by using each of the compounds of the present invention obtained in Examples 1 to 3, and Comparative Compounds 1 and 2 as a raw material for chemical vapor deposition by an ALD method under the following conditions with the apparatus illustrated in FIG. 1. The resultant thin-film was subjected to thickness measurement by an X-ray reflectivity method, identification of the compound of the thin-film by an X-ray diffraction method, and measurement of a carbon content in the thin-film by X-ray photoelectron spectroscopy. The results are shown in Table 2.

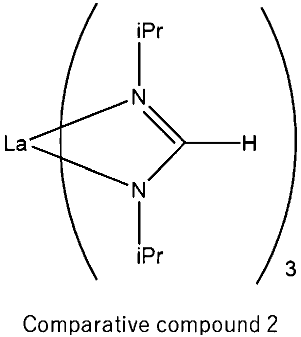

### [Examples 4 to 6, and Comparative Examples 1 and 2] Production of Lanthanum Oxide Thin-film by ALD Method

### (Conditions)

Reaction temperature (substrate temperature): 200°C, reactive gas: water vapor

### (Steps)

A series of steps consisting of the following steps (1) to (4) was defined as one cycle, and the cycle was repeated 50 times.
(1) Vapor of a raw material for chemical vapor deposition vaporized under the conditions of a raw material container heating temperature of 150°C and a raw material container inner pressure of 100 Pa is introduced and deposited at a system pressure of 100 Pa for 10 seconds (raw material introduction step and precursor thin-film formation step).
(2) An unreacted raw material is removed by purging with argon for 30 seconds (evacuation step).
(3) A reactive gas is introduced to be reacted at a system pressure of 100 Pa for 0.1 second (thin-film formation step).
(4) An unreacted raw material is removed by purging with argon for 120 seconds (evacuation step).

**Table 2**

| | Raw material for chemical vapor deposition | Thickness of thin-film | Compound of thin-film | Carbon content in thin-film |
|---|---|---|---|---|
| Example 4 | Compound No. 33 | 3.9 nm | Lanthanum oxide | Unable to be detected*¹ |
| Example 5 | Compound No. 37 | 4.0 nm | Lanthanum oxide | Unable to be detected*¹ |
| Example 6 | Compound No. 38 | 3.8 nm | Lanthanum oxide | Unable to be detected*¹ |
| Comparative Example 1 | Comparative Compound 1 | 2.9 nm | Lanthanum oxide | 4 atm% |
| Comparative Example 2 | Comparative Compound 2 | 2.8 nm | Lanthanum oxide | 4 atm% |

| | | | | |
|---|---|---|---|---|
| *1: The detection limit is 0.1 atm%. | | | | |

While the carbon content in the lanthanum oxide thin-film obtained by the ALD method was 4 atm% in each of Comparative Examples 1 and 2, the carbon content was less than 0.1 atm% that was the detection limit in each of Examples 4 to 6. That is, the foregoing indicated that a high-quality lanthanum oxide thin-film was obtained through use of the compound of the present invention. In addition, while the thickness of the resultant thin-film was 2.9 nm or less in each of Comparative Examples 1 and 2, the thickness was 3.8 nm or more in each of Examples 4 to 6. Thus, the lanthanum oxide thin-film was obtained with high productivity through use of the compound of the present invention. In particular, the thickness of the resultant thin-film was 3.9 nm or more in each of Examples 4 and 5. Thus, the lanthanum oxide thin-film was obtained with higher productivity. In particular, in Example 5, the thickness of the resultant thin-film was 4.0 nm, and thus the lanthanum oxide thin-film was obtained with particularly high productivity.

As described above, the compound of the present invention was a compound having a high vapor pressure and a low melting point, and was able to provide a high-quality thin-film with high productivity when the compound was used as a raw material for chemical vapor deposition. Thus, it was indicated that the compound of the present invention was excellent as a raw material for chemical vapor deposition. Of those, Compounds No. 33 and No. 37 were compounds each having a high vapor pressure and a low melting point, and were each able to provide a thin-film with higher productivity when the compound was used as a raw material for chemical vapor deposition. Thus, it was indicated that Compounds No. 33 and No. 37 were each excellent as a raw material for chemical vapor deposition. In particular, Compound No. 37 was a compound having a high vapor pressure and a particularly low melting point, and was able to provide a thin-film with particularly high productivity when the compound was used as a raw material for chemical vapor deposition. Thus, it was indicated that Compound No. 37 was particularly excellent as a raw material for chemical vapor deposition.

### Reference Signs List

- 100: film formation chamber

- 101: raw material container
- 102: vaporization chamber
- 103: heater
- 104: mass flow controller (MFC)
- 105: vacuum pump
- 106: cooling trap
- 107: reactive gas
- 108: purge gas
- 109: exhaust gas
- 110: carrier gas
- 111: carrier gas
- 112: automatic pressure controller
- 113: radio frequency (RF) power source
- 114: RF matching system

## Claims

1. A compound represented by the following general formula (1) : 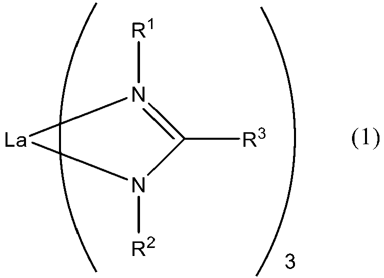 where R¹ and R² each independently represent an alkyl group having 1 to 8 carbon atoms, and R³ represents a hydrogen atom or an alkyl group having 1 to 5 carbon atoms, provided that R¹ and R² represent different groups.

2. The compound according to claim 1, wherein R¹ and R² each represent a branched alkyl group having 3 to 6 carbon atoms.

3. A thin-film forming raw material, comprising the compound of claim 1.

4. A thin-film formed from the thin-film forming raw material of claim 3.

5. A method of producing a thin-film, comprising forming a thin-film containing a lanthanum atom on a surface of a substrate through use of the thin-film forming raw material of claim 3.

6. The method of producing a thin-film according to claim 5, comprising:
a raw material introduction step of introducing a raw material gas obtained by vaporizing the thin-film forming raw material into a film formation chamber having a substrate set therein; and
a thin-film formation step of subjecting the compound represented by the general formula (1) to heating and/or plasmatization to form the thin-film containing the lanthanum atom on the surface of the substrate.

7. The method of producing a thin-film according to claim 6, further comprising, between the raw material introduction step and the thin-film formation step, a precursor thin-film formation step of forming a precursor thin-film on the surface of the substrate through use of the thin-film forming raw material,
wherein the thin-film formation step is a step of subjecting the compound represented by the general formula (1) to heating and/or plasmatization in the presence of a reactive gas.

8. A use of the compound of claim 1 for forming a thin-film.
